# DEMANDE DE BREVET EUROPEEN

(11) **EP 1 491 182 A2**
(43) Date de publication de la demande: **29.12.2004**
(21) Numéro de dépôt: 04291237.8
(22) Date de dépôt: 14.05.2004
(51) Int. Cl.: A61K 7/32

(54) **Composition cosmétique déodorante comprenant au moins un polymère semi-cristallin**

(30) Priorité: 27.06.2003 FR 0307804
(71) Demandeur: L'OREAL, 75008 Paris (FR)
(72) Inventeur: Prud'Homme, Estelle, 75018 Paris (FR); Douin, Véronique, 75017 Paris (FR)
(74) Mandataire: Miszputen, Laurent

(57) **Abrégé**

L'invention concerne une composition déodorante comprenant un support physiologiquement acceptable du type émulsion eau-dans-huile, caractérisée par le fait qu'elle contient au moins un actif anti-transpirant et au moins un polymère semi-cristallin ayant un point de fusion supérieur ou égal à 30 °C

L'invention concerne également l'utilisation de ladite composition pour la fabrication de produits cosmétiques destinés à l'application topique humaine, en particulier les produits déodorants ainsi qu'un procédé pour traiter la transpiration humaine et notamment les odeurs corporelles liées à la transpiration humaine.

Cette composition peut constituer notamment une crème cosmétique, ayant une texture très agréable à l'application sur la peau.

## Description

L'invention concerne une composition déodorante comprenant un support physiologiquement acceptable du type émulsion eau-dans-huile, caractérisée par le fait qu'elle contient au moins un actif anti-transpirant et au moins un polymère semi-cristallin ayant un point de fusion supérieur ou égal à 30 °C.

L'invention concerne également l'utilisation de ladite composition pour la fabrication de produits cosmétiques destinés à l'application topique humaine, en particulier les produits déodorants ainsi qu'un procédé pour traiter la transpiration humaine et notamment les odeurs corporelles liées à la transpiration humaine.

Dans le domaine cosmétique, il est bien connu d'utiliser en application topique, des produits déodorants contenant des substances actives de type antitranspirant ou de type bactéricide pour diminuer voire supprimer les odeurs corporelles liées à la transpiration généralement désagréables.

Les substances anti-transpirantes ont pour effet de limiter le flux sudoral. Elles sont généralement constituées de sels d'aluminium.

Les produits déodorants sous forme de crèmes à base d'actifs anti-transpirants sont particulièrement recherchés dans la mesure où ils sont plus malléables et déformables que les compositions solides, faciles à étaler sur la peau et de texture agréable. Parmi les crèmes proposées actuellement sur le marché des déodorants, on distingue (1) les émulsions huile-dans-eau (H/E) dites "directes" comportant une phase huileuse dispersée dans une phase aqueuse ; (2) les crèmes anhydres qui sont généralement à base de cires ou à base d'épaississants de phase grasse et (3) les gels du type émulsion eau-dans-silicone comportant une phase aqueuse dispersée dans une phase huileuse à base de silicone.

Les crèmes déodorantes sous forme d'émulsion huile-dans-eau présentent l'inconvénient de sécher très lentement après application et de conduire à un effet collant désagréable sur la peau.

Les crèmes déodorantes anhydres ont tendance à donner un effet gras sur la peau et ne permettent pas en général de produire un effet frais sur la peau.

Les gels du type émulsion eau-dans-silicone présentent l'inconvénient d'apporter lors de l'application sur la peau, un étalement assez huileux du fait que la phase huileuse est la phase externe et un toucher collant lors du séchage de l'émulsion.

Il subsiste donc le besoin de rechercher une nouvelle crème déodorante, ne présentant pas les inconvénients rencontrés avec celles connues jusqu'à présent et donnant un dépôt non-gras, non collant et frais, présentant un temps de séchage rapide après application.

De façon surprenante, la demanderesse a trouvé que l'utilisation d'au moins un polymère semi-cristallin ayant un point de fusion supérieur ou égal à 30 °C dans un support du type émulsion eau-dans-huile comprenant au moins un actif anti-transpirant conduisait à une crème déodorante de texture légère et fondante, donnant un dépôt non-gras, non collant et frais et ayant une bonne efficacité contre les odeurs corporelles en particulier les odeurs axillaires.

On entend ici par « support physiologiquement acceptable », un support non toxique et susceptible d'être appliqué sur la peau.

Par "polymères", on entend au sens de l'invention des composés comportant au moins 2 motifs de répétition, de préférence au moins 3 motifs de répétition et plus spécialement au moins 10 motifs de répétition.

Par "polymère semi-cristallin", on entend au sens de l'invention, des polymères comportant une partie cristallisable, chaîne pendante cristallisable ou séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe.

Par "séquence", on entend généralement au moins 5 motifs de répétition identiques. La ou les séquences cristallisables sont alors de nature chimique différente de la ou des séquences amorphes.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitifs du polymère. Avantageusement, la "chaîne pendante cristallisable" peut être une chaîne comportant au moins 6 atomes de carbone.

Le polymère semi-cristallin selon l'invention a une température de fusion supérieure ou égale à 30 °C, de préférence allant de 30 °C à 80 °C), et plus préférentiellement allant de 30 °C à 70 °C. Cette température de fusion est une température de changement d'état du premier ordre. Cette température de fusion peut être mesurée par toute méthode connue et en particulier à l'aide d'un calorimètre à balayage différentiel (D.S.C).

De façon avantageuse, le ou les polymères semi-cristallins auxquels s'applique l'invention présentent une masse moléculaire moyenne en nombre supérieure ou égale à 1 000.

De façon avantageuse, le ou les polymères semi-cristallins de la composition de l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. En outre, ils ne comportent pas de squelette polysaccharidique. De façon générale, les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins.

Selon l'invention, les polymères semi-cristallins sont choisis parmi les copolymères séquencés comportant au moins une séquence cristallisable et au moins une séquence amorphe, les homopolymères et les copolymères portant au moins une chaîne latérale cristallisable par motif de répétition, leurs mélanges.

Les polymères semi-cristallins utilisables dans l'invention sont en particulier :
- les copolymères séquencés de polyoléfines à cristallisation contrôlée, notamment ceux dont les monomères sont décrits dans EP-A-0 951 897.
- les polycondensats et notamment de type polyester, aliphatique ou aromatique
   ou copolyester aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré(s) tels que décrits dans le document WO-A-01/19333, et leurs mélanges. Dans ces deux derniers cas, la ou les chaînes latérales ou séquences cristallisables sont hydrophobes.

### A) Polymères semi-cristallins à chaînes latérales cristallisables

On peut citer en particulier ceux définis dans le document US-A-5156911 et WO-A-01/19333. Ce sont des homopolymères ou copolymères comportant de 50 à 100 % en poids de motifs résultant de la polymérisation d'un ou plusieurs monomères porteurs de chaîne latérale hydrophobe cristallisable.

Ces homo- ou co-polymères sont de toute nature du moment qu'ils présentent les conditions indiquées précédemment.
Ils peuvent résulter :
- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

D'une façon générale, ces polymères sont choisis notamment parmi les homopolymères et copolymères résultant de la polymérisation d'au moins un monomère à chaîne(s) cristallisable(s) qui peut être représenté par la formule X : 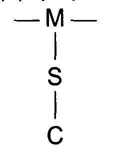 avec M représentant un atome du squelette polymérique, S représentant un espaceur, C représentant un groupe cristallisable.

Les chaînes « -S-C » cristallisables peuvent être aliphatiques ou aromatiques, éventuellement fluorées ou perfluorées. « S » représente notamment un groupe (CH₂)ₙ ou (CH₂CH₂O)ₙ ou (CH₂O), linéaire ou ramifié ou cyclique, avec n entier allant de 0 à 22. De préférence « S » est un groupe linéaire. De préférence, « S » et « C » sont différents.

Lorsque les chaînes «-S-C» cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyle en C₁₄-C₂₄. Lorsqu'il s'agit de chaînes alkyle fluorées ou perfluorées, elles comportent au moins 6 atomes de carbone fluorés et notamment au moins 11 atomes de carbone dont au moins 6 atomes de carbone sont fluorés.

Comme exemples de polymères ou copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en C₁₄-C₂₄, les (méth)acrylates de perfluoroalkyle avec un groupe alkyle perfluoro en C₁₁-C₁₅, les N-alkyl (méth)acrylamides avec le groupe alkyle en C₁₄ à C₂₄ avec ou sans atome de fluor, les esters vinyliques à chaînes alkyle ou perfluoro (alkyle) avec le groupe alkyle en C₁₄ à C₂₄ (avec au moins 6 atomes de fluor pour une chaîne perfluoro alkyle), les éthers vinyliques à chaînes alkyle ou perfluoro (alkyle) avec le groupe alkyle en C₁₄ à C₂₄ et au moins 6 atomes de fluor pour une chaîne perfluoro alkyle, les alpha-oléfines en C₁₄ à C₂₄ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y ou Z résultant de la copolymérisation :
α) de Y qui est un monomère polaire ou non polaire ou un mélange des deux : . Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un N,N-dialkyl(méth)acrylamide comme par exemple le N,N-diisopropylacrylamide ou la N-vinyl-pyrrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
   Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou styrène substitué par un groupe alkyle en C₁ à C₁₀, comme l'α-méthylstyrène, un macromonomère du type polyorganosiloxane à insaturation vinylique.
   Par "alkyle», on entend au sens de l'invention un groupement saturé notamment en C₈ à C₂₄, sauf mention exprès, et mieux en C₁₄ à C₂₄.
β) de Z qui est un monomère polaire ou un mélange de monomères polaires. Dans ce cas, Z a la même définition que le "Y polaire" défini ci-dessus. De préférence, les polymères semi-cristallins à chaîne latérale cristallisable sont des homopolymères d'alkyl(méth)acrylate ou d'alkyl(méth)acrylamide avec un groupe alkyle tel que défini ci-dessus, et notamment en C₁₄-C₂₄, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

### B) Les polymères portant dans le squelette au moins une séquence cristallisable

Ces polymères sont notamment des copolymères séquencés constitués d'au moins 2 séquences de nature chimique différente dont l'une est cristallisable.
- On peut utiliser les polymères séquencés définis dans le brevet US-A-5156 911 ; les copolymères séquencés d'oléfine ou de cyclooléfine à chaîne cristallisable comme ceux issus de la polymérisation séquencée de : cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8,8a-octahydronaphtalène, dicyclopentadiène ou leurs mélanges, avec l'éthylène, le propylène, le 1-butène, le 3-méthyl-1-butène, le 1-hexène, le 4-méthyl-1-pentène, le 1-octène, le 1-décène, le 1-éicosène ou leurs mélanges, et en particulier les copoly(éthylène/norbornène) blocs et les terpolymères (éthylène/propylène/éthylidène-norbornène) blocs. On peut aussi utiliser ceux résultant de la copolymérisation séquencée d'au moins 2 α-oléfines en C₂-C₁₆ et mieux en C₂-C₁₂ et encore mieux en C4-C12 tels que ceux cités précédemment et en particulier les bipolymères séquencés d'éthylène et d'1-octène.

Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
Séquence cristallisable par nature : a) polyester comme les poly(alkylène téréphtalate), b) polyoléfine comme les polyéthylènes ou polypropylènes.
Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe distinctes, on peut citer :
α) les copolymères séquencés poly(ε-caprolactone)-b-poly(butadiène), utilisés de préférence hydrogénés, tels que ceux décrits dans l'article "Melting behavior of poly(ε-caprolactone)-block-polybutadiene copolymers" de S. Nojima, Macromolécules, 32, 3727-3734 (1999).
β) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).
γ) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et " Polymer aggregates with crystalline cores : the system poly(ethylene)-poly(ethylene-propylene)" de P. Richter et al., Macromolécules, 30, 1053-1068 (1997).
δ) les copolymères séquencés poly(éthylène)-b-poly(éthyléthylène) cités dans l'article général "Crystallization in block copolymers" de I.W. Hamley, Advances in Polymer Science, vol 148, 113-137 (1999).

Les polymères semi-cristallins de la composition de l'invention peuvent être ou non réticulés en partie du moment que le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.

De préférence, les polymères semi-cristallins de la composition selon l'invention sont non réticulés.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en C₁₄ à C₂₄, les (méth)acrylates de perfluoroalkyle en C₁₁ à C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alphaoléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ éventuellement fluoré, qui peut être représenté par la formule suivante : 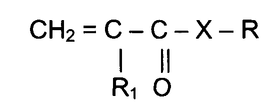 dans laquelle R₁ est H ou CH₃, R représente un groupe alkyle en C1-C10 éventuellement fluoré et X représente O, NH ou NR₂, où R₂ représente un groupe alkyle en C₁-C₁₀ éventuellement fluoré.

Avantageusement, le polymère semi-cristallin présent dans la composition selon l'invention n'est pas une polycaprolactone.

A titre d'exemple particulier de polymère semi-cristallin structurant utilisable dans la composition selon l'invention, on peut citer les produits Intelimer® de la société Landec décrits dans la brochure "Intelimer ® polymers", Landec IP22 (Rev. 4-97). Ces polymères sont sous forme solide à température ambiante (25°C). Ils sont porteurs de chaînes latérales cristallisables et présentent la formule X précédente.

Les polymères semi-cristallins peuvent être notamment :
- ceux décrits dans les exemples 3, 4, 5, 7, 9, 13 du brevet US-A-5156911 à groupement -COOH, résultant de la copolymérisation d'acide acrylique et d'alkyl(méth)acrylate en C₅ à C₁₆ et plus particulièrement de la copolymérisation :
   d'acide acrylique, d'hexadécylacrylate et d'isodécylacrylate dans un rapport pondéral 1/16/3,
   d'acide acrylique et de pentadécylacrylate dans un rapport pondéral 1/19,
   d'acide acrylique, d'hexadécylacrylate, éthylacrylate dans un rapport pondéral 2,5/76,5/20,
   d'acide acrylique, d'hexadécylacrylate et de méthylacrylate dans un rapport pondéral 5/85/10,
   d'acide acrylique et de octadécylméthacrylate dans un rapport pondéral 2,5/97,5 ,
   d'hexadécylacrylate, de monométhyl éther de méthacrylate polyéthylèneglycol à 8 motifs d'éthylèneglycol, et d'acide acrylique dans un rapport pondéral 8,5/1/0,5.

On peut aussi utiliser le «Structure O» de National Starch tel que celui décrit dans le document US-A-5736125 de point de fusion 44°C ainsi que les polymères semi-cristallins à chaînes pendantes cristallisables comportant des groupements fluorés tels que décrits dans les exemples 1, 4, 6, 7 et 8 du document WO-A-01/19333.

On peut encore utiliser les polymères semi-cristallins de bas point de fusion obtenus par copolymérisation d'acrylate de stéaryle et d'acide acrylique ou de NVP tels que décrits dans le document US-A-5519063 ou EP-A-550745 et plus spécialement ceux décrits dans les exemples 1 et 2, ci-après, de préparation de polymère, de température de fusion respectivement de 40°C et 38°C.

On peut aussi utiliser les polymères semi-cristallins obtenus par copolymérisation de l'acrylate de béhényle et de l'acide acrylique ou de NVP tels que décrits dans les documents US-A-5519063 et EP-A-550745.

De préférence, les polymères semi-cristallins à bas point de fusion et/ou ceux à haut point de fusion ne comportent pas de groupement carboxylique.

Selon un mode plus particulier de réalisation de l'invention, le polymère est issu d'un monomère à chaîne cristallisable choisi parmi les (méth)acrylates d'alkyle saturés en C14 à C22 et encore plus particulièrement les poly(acrylate de stéaryle) ou les poly(acrylate de béhényle). Par exemple, on choisit le produit Intelimer® IPA 13-1 de la société Landec, qui est un polyacrylate de stéaryle de poids moléculaire d'environ 145 000 et dont la température de fusion est égale à 49°C.

Le polymère semi-cristallin peut être présent dans la composition en une teneur allant de 0,1 % à 50 % en poids, par rapport au poids total de la composition, de préférence de 0,1 % à 20 % en poids, et mieux de 0,1 % à 15 % en poids par rapport au poids total de la composition.

Le ou les polymères semi-cristallins utilisés et la quantité de ces polymères sont choisis selon la finalité de la composition désirée et en fonction du mode d'application particulier envisagé (tube, roll-on ou stick-grille).

Parmi les actifs anti-transpirants utilisables selon l'invention, on peut citer les sels d'aluminium et/ou de zirconium ; les complexes d'hydroxychlorure de zirconium et d'hydroxychlorure d'aluminium avec un acide aminé tels que ceux décrits dans le brevet US-3792068 communément connus sous l'appellation "ZAG complexes (lorsque l'acide aminé est la glycine )".

Parmi les sels d'aluminium, on peut citer en particulier le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

Parmi les sels d'aluminium et de zirconium, on peut citer en particulier l'aluminium zirconium octachlorohydrate, l'aluminium zirconium pentachlorohydrate, l'aluminium zirconium tetrachlorohydrate, l'aluminium zirconium trichlorohydrate.

Les complexes d'hydroxychlorure de zirconium et d'hydroxychlorure d'aluminium avec un acide aminé sont généralement connus sous l'appellation ZAG (lorsque l'acide aminé est la glycine). Parmi ces produits on peut citer les complexes aluminium zirconium octachlorohydrex glycine, aluminium zirconium pentachlorohydrex glycine, aluminium zirconium tetrachlorohydrex glycine et aluminium zirconium trichlorohydrex glycine.

On utilisera plus particulièrement le chlorhydrate d'aluminium sous forme activée ou non activée.

Le ou les actifs anti-transpirants sont présents de préférence dans les compositions selon l'invention dans des concentrations pondérales (ramenées à l'ensemble de la composition) allant de 0,1 à 40%, et plus particulièrement, allant de 0,5 à 25%.

Les compositions selon l'invention peuvent contenir en plus d'autres actifs déodorants.

Au sens de la présente invention, on entend par "actif déodorant" toute substance capable de réduire ou supprimer le flux sudoral et/ou d'absorber la sueur humaine et/ou de masquer, absorber, améliorer ou réduire l'odeur désagréable résultant de la décomposition de la sueur humaine par des bactéries.

Les actifs déodorants additionnels peuvent être des agents bactériostatiques ou des agents bactéricides comme le 2,4,4'-trichloro-2'-hydroxydiphényléther (®Triclosan), le 2,4-dichloro-2'-hydroxydiphényléther, le 3',4',5'-trichlorosalicylanilide, la 1-(3',4'-dichlorophenyl)-3-(4'-chlorophenyl)urée (®Triclocarban) ou le 3,7,11-triméthyldodéca-2,5,10-triénol (®Farnesol) ; les sels d'ammonium quaternaires comme les sels de cetyltrimethylammonium, les sels de cétylpyridinium

Parmi les actifs déodorants additionnels, on peut aussi citer également les sels de zinc comme le salicylate de zinc, le sulfate de zinc, le chlorure de zinc, le lactate de zinc, le phénolsulfonate de zinc ; la chlorhexidine et ses sels; le bicarbonate de sodium ; le monocaprate de diglycérol, le monolaurate de diglycérol , monolaurate de glycérol ; les sels de polyhexaméthylène biguanide.

Les actifs déodorants additionnels peuvent être présents dans la composition selon l'invention à raison d'environ 0,001 à 40% en poids par rapport à la composition totale, et de préférence à raison d'environ 0,1 à 25% en poids.

### Phase huileuse

Par "phase huileuse", au sens de la demande, on entend une phase grasse liquide à température ambiante (25°C) et pression atmosphérique (760 mm de Hg), composée notamment d'un ou plusieurs corps gras liquides à température ambiante, appelés aussi huiles, compatibles entre eux. Cette phase huileuse est macroscopiquement homogène.

Les compositions selon l'invention étant sous forme d'émulsion E/H, la phase huileuse gélifiée par le polymère semi-cristallin constitue la phase continue de l'émulsion. Cette phase huileuse peut être présente en une quantité allant par exemple de 10 à 95 % en poids, de préférence de 10 à 80 % en poids, mieux de 15 à 70 % en poids et encore mieux de 20 à 60 % en poids par rapport au poids total de la composition.

Cette phase huileuse contient au moins une huile, notamment une huile cosmétique, et elle peut contenir plusieurs huiles et éventuellement un ou plusieurs autres corps gras.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R₁COOR₂ et R₁OR₂ dans laquelle R₁ représente le reste d'un acide gras comportant de 8 à 29 atomes de carbone, et R₂ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles telles que les cyclopolydiméthylsiloxanes (cyclométhicones) comme la cyclohexasiloxane (ou cyclohexamethicone) et le cyclopentadiméthylsiloxane (ou cyclopentamethicone) et leurs mélanges ;
   ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste d'huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires ; les gommes telles que les gommes de silicone (diméthiconol) ; les résines de silicone telles que la trifluorométhyl-C₁-C₄-alkyldimethicone et la trifluoropropyldimethicone ; les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries ; ainsi que les élastomères de silicone comportant une ou plusieurs chaînes oxyalkylénés et notamment oxyéthylénés, tels que le produit commercialisé sous la dénomination « KSG 21 » par la société Shin-Etsu ; et leurs mélanges.

Ces corps gras peuvent être choisis de manière variée par l'homme du métier afin de préparer une composition ayant les propriétés, par exemple de consistance ou de texture, souhaitées.

### Emulsionnant

Les compositions selon l'invention contiennent en général au moins un émulsionnant eau-dans-huile.

L'émulsionnant de la composition selon l'invention a une valeur de HLB (hydrophile-lipophile balance) allant de 1 à 8.

L'émulsionnant peut être choisi par exemple parmi les émulsionnants siliconés, les alkylpolyglycosides (APG), les émulsionnants non ioniques dérivés d'acide gras et de polyol, et leurs mélanges.

Comme émulsionnants siliconés pouvant entrer dans la composition selon l'invention, on peut citer par exemple les dimethicone copolyols et les alkyl diméthicone copolyols. Comme dimethicone copolyol, on peut citer par exemple le mélange de dimethicone copolyol et de dimethicone (polydiméthylsiloxane) (10/90) commercialisé par la société Dow Corning sous la dénomination DC3225C. Selon un mode préféré de réalisation de l'invention, on utilise comme émulsionnant siliconé un alkyl diméthicone copolyol ayant un radical alkyle comportant de 10 à 22 atomes de carbone, tel que le cétyl diméthicone copolyol comme le produit commercialisé sous la dénomination Abil EM-90 par la société Goldschmidt et comme le mélange de cétyl diméthicone copolyol, d'isostearate de polyglycérole (4 moles) et de laurate d'hexyle vendu sous la dénomination ABIL WE 09 par la société Goldschmidt ; le lauryl diméthicone copolyol et par exemple le mélange d'environ 91 % de lauryl diméthicone copolyol et d'environ 9 % d'alcool isostéarylique, commercialisé sous la dénomination Q2-5200 par la société Dow Corning, et leurs mélanges.

Comme émulsionnants non ioniques dérivés d'acide gras et de polyol, on peut utiliser notamment les esters d'acide gras et de polyol, l'acide gras ayant notamment une chaîne alkyle en C₈-C₂₄, et les polyols étant par exemple le glycérol et le sorbitan. Comme esters d'acide gras et de polyol, on peut citer notamment les esters d'acide isostéarique et de polyols, les esters d'acide stéarique et de polyols, et leurs mélanges, en particulier les esters d'acide isostéarique et de glycérol et/ou de sorbitan, par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, le mélange d'isostéarate de sorbitan et d'isostéarate de polyglycérol (3 moles) commercialisé sous la dénomination Arlacel 1690 par la société Unigema. et leurs mélanges.

L'émulsionnant peut être choisi aussi parmi les alkylpolyglycosides ayant un HLB inférieur à 7, par exemple ceux représentés par la formule générale (I) suivante :

R-O-(G)x (I)

dans laquelle R représente un radical alkyle ramifié et/ou insaturé, comportant de 14 à 24 atomes de carbone, G représente un sucre réduit comportant de 5 à 6 atomes de carbone, et x désigne une valeur allant de 1 à 10 et de préférence de 1 à 4, et G désigne notamment le glucose, le fructose ou le galactose.

Le radical alkyle insaturé peut comprendre une ou plusieurs insaturations éthyléniques, et en particulier une ou deux insaturations éthyléniques.

Comme alkylpolyglycosides de ce type, on peut citer les alkylpolyglucosides (G=glucose dans la formule (I)), et notamment les composés de formule (I) dans laquelle R représente plus particulièrement un radical oléyle (radical insaturé en C₁₈) ou isostéaryle (radical saturé en C18), G désigne le glucose, x est une valeur allant de 1 à 2, notamment l'isostéaryl-glucoside, l'oléyl-glucoside et leurs mélanges. Cet alkylpolyglucoside peut être utilisé en mélange avec un co-émulsionnant, plus spécialement avec un alcool gras et notamment un alcool gras ayant la même chaîne grasse que celle de l'alkylpolyglucoside, c'est-à-dire comportant de 14 à 24 atomes de carbone et ayant une chaîne ramifiée et/ou insaturée, et par exemple l'alcool isostéarylique quand l'alkylpolyglucoside est l'isostéaryl-glucoside, et l'alcool oléylique quand l'alkylpolyglucoside est l'oleyl-glucoside, éventuellement sous forme d'une composition autoémulsionnante, comme décrit par exemple dans le document WO-A-92/06778. On peut utiliser par exemple le mélange d'isostéaryl-glucoside et d'alcool isostéarylique, commercialisé sous la dénomination Montanov WO 18 par la société SEPPIC.

L'émulsionnant est de préférence utilisé en une quantité en matière active allant par exemple de 0,1 à 20 % et de préférence de 0,5 à 10 % en poids par rapport au poids total de la composition.

L'émulsionnant est généralement introduit dans la phase huileuse de l'émulsion.

La phase aqueuse de la composition de l'invention représente généralement de 5 à 90 % en poids et de préférence de 20 à 60 % en poids par rapport au poids total de la composition. Elle peut contenir, outre l'eau, des solvants tels que les alcools primaires comportant de 1 à 6 atomes de carbone comme l'éthanol, ou les polyols comme le butylène glycol, la glycérine, le sorbitol, l'hexylène glycol, le propylène glycol et l'isoprène glycol, ou des sucres comme le glucose, le fructose. Le ou les solvants peuvent être présents en une quantité allant de 0,1 à 30 % en poids par rapport au poids total de la composition.

De manière préférentielle, les compositions déodorantes selon l'invention contiendront en plus une poudre organique.

On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m3), 551 DE 20 (granulométrie d'environ 30 µm et masse volumique 65 kg/m3), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyllysine commercialisée sous la dénomination AMIHOPE LL-11 par la Société Ajinomoto ; les particules de microdispersion de cire, qui ont de préférence des dimensions moyennes inférieures à 1 µm et notamment allant de 0,02 µm à 1 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges. La ou les poudres organiques peuvent être présentes par exemple en une quantité allant de 0,1 à 20 % en poids et de préférence de 0,5 à 10 % en poids par rapport au poids total de la composition.

Selon un mode préféré de réalisation de l'invention, la ou les poudres organiques sont ajoutées à la composition après mélange des phases aqueuse et huileuse.

La composition selon l'invention peut encore contenir d'autres ingrédients bien connus dans le domaine des produits cosmétiques déodorants, dont on peut citer par exemple, des agents apaisants, des parfums, des conservateurs, des agents antioxydants, des séquestrants, des gélifiants, des agents de suspension tels que les bentonites et les hectorites, des émollients, des actifs lipophiles ou hydrophiles et leurs mélanges. Ces additifs peuvent être présents dans la composition selon les quantités généralement utilisées dans le domaine cosmétique et dermatologique et notamment à raison de 0,01 à 50% du poids total de la composition et mieux de 0,1 à 20%. L'eau peut représenter jusqu'à 90 % du poids total de la composition.

Comme actifs, on peut citer par exemple les hydratants et par exemple les hydrolysats de protéines et les polyols tels que la glycérine, les glycols comme les polyéthylène glycols, et les dérivés de sucre ; les extraits naturels ; les oligomères procyanidoliques ; les vitamines comme la vitamine E (tocophérol) et ses dérivés (par exemple acétate), la vitamine A (rétinol) et ses dérivés (par exemple palmitate de rétinyle), la vitamine C (acide ascorbique) et ses dérivés (par exemple palmitate d'ascorbyle), les dérivés de ces vitamines étant notamment des esters dont le palmitate et l'acétate ; les acides gras essentiels ; les sphingolipides et céramides ; les filtres solaires ; les bêta-hydroxyacides tels que l'acide salicylique et ses dérivés ; les alpha-hydroxyacides tels que l'acide lactique, l'acide citrique et l'acide glycolique ; les rétinoïdes tels que le rétinol et ses esters, le rétinal, les caroténoïdes ; et leurs mélanges.

Comme gélifiants, on peut utiliser notamment les gélifiants hydrophiles tels que les polymères carboxyvinyliques, comme les carbomers ; les polyacrylamides et les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Clariant sous la dénomination commerciale « Hostacerin AMPS » (nom CTFA : ammonium polyacryldimethyltauramide); ou le copolymère acrylamide /acrylamido-2-methylpropane sulfonate de sodium en émulsion inverse à 40 % dans le polysorbate, commercialisé sous la dénomination SIMULGEL 600 par la société SEPPIC ; les polysaccharides tels que la gomme de xanthane ; et leurs mélanges.

Ces émollients peuvent être choisis parmi des produits du type silicone volatile, des silicones non-volatiles et d'autres émollients non-volatiles.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones D4 ou D5.
Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 200 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.

Parmi les émollients non-volatils utilisables dans la présente invention, on peut citer par exemple : les dérivés hydrocarbonés, les huiles minérales, les alcools gras, les esters d'alcools en C₃-C₁₈ avec des acides en C₃-C₁₈, les esters de l'acide benzoïque avec des alcools en C₁₂-C₁₈ et leurs mélanges, des polyols en C₂-C₆ choisis de préférence parmi le glycérol, le propylèneglycol ou le sorbitol, les polymères de polyalkylène glycol.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

Les compositions selon l'invention peuvent être fabriquées par les procédés connus, généralement utilisés dans le domaine des émulsions du type eau-dans-huile. Elle peuvent être fabriquées par un procédé qui consiste à chauffer le polymère au moins à sa température de fusion (PF), à y ajouter le ou les émulsionnants et autres constituants de la phase huileuse, à préparer la phase aqueuse à chaud (70 à 80°C environ) et à introduire la phase aqueuse dans la phase huileuse sous agitation, puis à ajouter la ou les poudres à chaud ou à froid.

Les compositions déodorantes selon l'invention peuvent se présenter selon leur viscosité sous forme de crèmes liquides (laits) ou épaisses distribuées en tubes, en roll-on ou sous forme de sticks-grilles.

D'une manière générale leur viscosité à 25°C mesurée avec un appareil du type Rhéomat (RM 180 commercialisé par la société Mettler) muni d'un système de mesure Contraves TV, à une vitesse de 200 tours/minutes et après 30 secondes de stabilisation varie de 0.2 à 7 Pa.s.

La présente invention a également pour objet un procédé cosmétique pour traiter la transpiration humaine, consistant à appliquer sur la surface axillaire une quantité efficace d'une composition telle que définie ci-dessus.

La présente invention a pour objet également un procédé cosmétique pour traiter les odeurs corporelles humaines, consistant à appliquer sur la surface axillaire une quantité efficace d'une composition telle que définie précédemment.

L'invention est illustrée plus en détail dans les exemples suivants. Les quantités sont données en pourcentage massique.

### I) Exemples de fabrication de polymères semi-cristallins

### Exemple A : Homopolymère de point de fusion de 48°C

Dans un réacteur d'1l muni d'une agitation centrale avec ancre, d'un réfrigérant et d'un thermomètre, on introduit 120g de Parléam que l'on chauffe de la température ambiante à 80°C en 45 min. A 80°C, on introduit en 2h le mélange C₁ suivant :
40g de cyclohexane + 4g de Trigonox 141 [ 2,5 bis (2-éthyl hexanoyl peroxy) - 2,5 - diméthyl hexane].
30 min après le début de la coulée du mélange C₁, on introduit en 1 h30 le mélange C₂ constitué de :
   200 g d'acrylate de stéaryle + 400 g de cyclohexane.

A la fin des deux coulées, on laisse agir 3h supplémentaires à 80°C puis on distille à pression atmosphérique la totalité du cyclohexane présent dans le milieu réactionnel.
On obtient alors le polymère à 60 % en poids en matière active dans le Parléam.

Sa masse moléculaire moyenne en poids est de l'ordre 20 000-30 000 et sa température de fusion Tf est de 48°C, mesurée par D.S.C.

### Exemple B : Homopolymère de point de fusion de 58°C

On applique le même mode opératoire que dans l'exemple A, sauf que l'on utilise de l'acrylate de béhényle en lieu et place de l'acrylate de stéaryle. Le polymère obtenu est à 60% en poids en matière active dans le Parléam. Sa masse moléculaire moyenne en poids est de 17 000-27 000 et sa température de fusion de 58°C.
). Par exemple, on choisit le produit Intelimer® IPA 13-1 de la société Landec, qui est un polyacrylate de stéaryle de poids moléculaire d'environ 145 000 et dont la température de fusion est égale à 49°C.

### II) Exemples 1 à 5 de crèmes antitranspirantes

| **Ingrédients** | **Exemple 1** | **Exemple 2** | **Exemple 3** | **Exemple 4** | **Exemple 5** |
|---|---|---|---|---|---|
| Cetyl PEG/PPG-10/1 dimethicone | 1,50 | 1,50 | 1,50 | 1,50 | 1,50 |
| Polyglyceryl 4 isostearate | 0,50 | 0,50 | 0,50 | 0,50 | 0,50 |
| Isohexadecane | 15,50 | 15,50 | 15,50 | 15,50 | 15,50 |
| Polyacrylate de stéaryle de PM d'environ 145 000 et de température de fusion 49°C (Intelimer® IPA 13-1 de la société Landec) | | | 1,50 | | |
| Homopolymère selon l'exemple B | 1,50 | 0,20 | - | 1,28 | 3,00 |
| Cyclohexasiloxane | 8,28 | | | 8.28 | |
| Huile de silicone | | 7,40 | 7,40 | | 6,15 |
| Expancel 551 | 1,00 | 0,75 | 0,25 | 1,00 | 0,50 |
| Aluminium chlorohydrate en solution aqueuse à 50% | 40,00 (20% en matière active) | 40,00 (20% en matière active) | 40,00 (20% en matière active) | 40,00 (20% en matière active) | 40,00 (20% en matière active) |
| Eau | 28,85 | 30,15 | 28,35 | 28,85 | 28,85 |
| Aluminium Starch octenyl succinate | 3,00 | | 4,00 | | 3,00 |
| Billes de polyéthylène | | | | 3,00 | |
| Orgasol 2002 EXD NAT COS | | 3,00 | | | |
| Parfum | 0,10 | 1,00 | 1,00 | 0,10 | 1,00 |
| Conditionnement approprié | Tube | Roll on | Tube | Tube | Stick grille |

### Mode de fabrication des compositions 1 à 5 :

Chaque phase est chauffée à chaud (>70°C), puis la phase aqueuse est dispersée dans la phase huileuse sous agitation. La dispersion est refroidie à 50°C, puis la poudre organique et le parfum sont rajoutés.

Les crèmes antitranspirantes ainsi obtenues présentent une sensation à l'étalement peu huileuse, non-collant et produisent une sensation de frais et non-gras.

## Revendications

1. Composition déodorante comportant un support physiologiquement acceptable du type émulsion eau-dans-huile, **caractérisée en ce qu'**elle contient au moins un actif anti-transpirant et au moins un polymère semi-cristallin ayant un point de fusion supérieur ou égal à 30 °C.

2. Composition selon la revendication 1, **caractérisée par le fait que** le polymère semi-cristallin a un point de fusion allant de 30 °C à 80 °C, de préférence allant de 30°C à 70°C.

3. Composition selon l'une des revendications précédentes, dans laquelle le polymère semi-cristallin présente une masse moléculaire moyenne en nombre supérieur ou égale à 1 000.

4. Composition selon l'une des revendications précédentes, **caractérisée en ce que** le polymère semi-cristallin a une masse moléculaire moyenne en nombre allant de 3 000 à 500 000 et mieux de 4 000 à 150 000.

5. Composition selon l'une des revendications précédentes, dans laquelle le polymère semi-cristallin comporte i) un squelette polymérique et ii) au moins une chaîne latérale cristallisable et/ou une séquence organique cristallisable faisant partie du squelette dudit polymère semi-cristallin.

6. Composition selon l'une des revendications précédentes, **caractérisée en ce que** le polymère semi-cristallin est choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

7. Composition selon l'une des revendications précédentes, **caractérisée en ce que** la séquence cristallisable est de nature différente de la séquence amorphe.

8. Composition selon l'une quelconque des revendications précédentes, **caractérisée en ce que** le polymère semi-cristallin a une chaîne organique cristallisable et/ou une séquence cristallisable représentant au moins 30 % et de préférence 40% du poids total du polymère.

9. Composition selon l'une des revendications précédentes, **caractérisée en ce que** le polymère semi-cristallin est choisi parmi :
- les copolymères séquencés de polyoléfines à cristallisation contrôlée,
- les polycondensats polyesters aliphatiques ou aromatiques et les copolyesters aliphatiques/aromatiques,
- les homo- ou co-polymères portant au moins une chaîne latérale cristallisable,
- leurs mélanges.

10. Composition selon l'une des revendications précédentes, **caractérisée en ce que** le polymère semi-cristallin est choisi parmi les homopolymères et copolymères comportant de 50 à 100 % en poids de motifs résultant de la polymérisation de un ou plusieurs monomères porteurs de chaîne(s) latérale(s) hydrophobe(s) cristallisable(s).

11. Composition selon l'une des revendications précédentes, **caractérisée en ce que** le polymère semi-cristallin est choisi parmi les homopolymères et copolymères séquencés résultant de la polymérisation d'au moins un monomère à séquence(s) amorphe(s) ou chaîne(s) latérale(s) cristallisable(s) par motif de répétition , leurs mélanges de formule X : 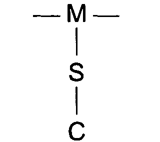 avec M représentant un atome du squelette polymérique, S représentant un espaceur, C représentant un groupe cristallisable et leurs mélanges, avec « S-C » représentant une chaîne alkyle à au moins 11 atomes de carbone, éventuellement fluorée ou perfluorée.

12. Composition selon l'une des revendications précédentes, **caractérisée en ce que** le polymère semi-cristallin est choisi parmi les polymères résultant de la polymérisation d'au moins un monomère choisi parmi l'acide acrylique, méthacrylique, crotonique, itaconique, maléique, l'anhydride maléique et leurs mélanges.

13. Composition selon l'une des revendications précédentes, **caractérisée en ce que** le polymère semi-cristallin est choisi parmi les homopolymères et copolymères résultant de la polymérisation d'au moins un monomère à séquence cristallisable choisis parmi les (méth)acrylates d'alkyle saturés en C₁₄-C₂₄, les (méth)acrylates de perfluoroalkyle en C₁₁-C₁₅, les N-alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, leurs mélanges.

14. Composition selon l'une quelconque des revendications précédentes, **caractérisée en ce que** le polymère semi-cristallin est choisi parmi les copolymères résultant de la polymérisation d'au moins un monomère à chaîne cristallisable choisi parmi les (méth)acrylates d'alkyle saturés en C₁₄ à C₂₄, les (méth)acrylates de perfluoroalkyle en C₁₁ à C₁₅, les N-alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaîne alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ éventuellement fluoré.

15. Composition selon l'une quelconque des revendications précédentes, **caractérisée par le fait que** le polymère semi-cristallin est choisi parmi les homopolymères d'alkyl(méth)acrylate ou d'alkyl(méth)acrylamide avec un groupe alkyle en C₁₄ à C₂₄, les copolymères de ces monomères avec un monomère hydrophile.

16. Composition selon l'une quelconque des revendications précédentes, **caractérisée** en par le fait que les polymères semi-cristallins sont des copolymères d'alkyl(méth)acrylate ou d'alkyl (méth)acrylamide avec un groupe alkyle en C₁₄ à C₂₄ avec un monomère hydrophile de nature différente de l'acide (méth)acrylique comme la N-vinylpyrrolidone ou l'hydroxyéthyl (méth)acrylate, et leurs mélanges.

17. Composition selon l'une quelconque des revendications précédentes, **caractérisée en ce que** le polymère semi-cristallin est issu d'un monomère à chaîne cristallisable choisi parmi les (méth)acrylates d'alkyle saturés en C₁₄ à C₂₂.

18. Composition selon la revendication 17, **caractérisée en ce que** le polymère semi-cristallin est choisi parmi les poly(acrylate de stéaryle) ou les poly(acrylate de béhényle).

19. Composition selon l'une des revendications précédentes, **caractérisée en ce que** le polymère semi-cristallin est présent en une teneur allant de 0,1 à 50 % en poids, par rapport au poids total de la composition, et mieux allant de 0,1 à 20 % en poids, et encore mieux allant de 0,1 % à 15 % en poids.

20. Composition selon l'une des revendications précédentes, où l'actif anti-transpirant est choisi parmi les sels d'aluminium et/ou de zirconium ; les complexes d'hydroxychlorure de zirconium et d'hydroxychlorure d'aluminium avec un acide aminé

21. Composition selon la revendication 20, où l'actif anti-transpirant est choisi parmi le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

22. Composition selon la revendication 20, où l'actif anti-transpirant est choisi parmi l'aluminium zirconium octachlorohydrate, l'aluminium zirconium pentachlorohydrate, l'aluminium zirconium tetrachlorohydrate, l'aluminium zirconium trichlorohydrate.

23. Composition selon la revendication 20, où l'actif anti-transpirant est choisi parmi les complexes d'hydroxychlorure de zirconium et d'hydroxychlorure d'aluminium avec la glycine (ZAG).

24. Composition selon la revendication 20, où l'actif anti-transpirant est choisi parmi les complexes aluminium zirconium octachlorohydrex glycine, aluminium zirconium pentachlorohydrex glycine, aluminium zirconium tetrachlorohydrex glycine et aluminium zirconium trichlorohydrex glycine.

25. Composition selon la revendication 20 ou 21, où l'actif anti-transpirant est le chlorhydrate d'aluminium sous forme activée ou non activée.

26. Composition selon l'une des revendications précédentes, **caractérisée en ce que** le ou les actifs anti-transpirants sont présents dans des concentrations pondérales (ramenées à l'ensemble de la composition) allant de 0,1 à 40%, et plus particulièrement, allant de 0,5 à 25%.

27. Composition selon l'une des revendications précédentes, **caractérisée en ce qu'**elle contient au moins un actif déodorant additionnel.

28. Composition selon l'une quelconque des revendications précédentes, **caractérisée en ce que** la phase huileuse représente de 10 à 95 % en poids et de préférence de 10 à 80 % en poids par rapport au poids total de la composition.

29. Composition selon l'une quelconque des revendications précédentes, **caractérisée en ce que** la phase huileuse contient au moins une huile choisie parmi les huiles hydrocarbonées d'origine animale, les huiles hydrocarbonées d'origine végétale, les esters et les éthers de synthèse, les hydrocarbures linéaires ou ramifiés, d'origine minérale ou synthétique, les alcools gras ayant de 8 à 26 atomes de carbone, les huiles fluorées partiellement hydrocarbonées et/ou siliconées, les huiles de silicone, et leurs mélanges.

30. Composition selon l'une quelconque des revendications précédentes, **caractérisée en ce qu'**elle contient au moins un l'émulsionnant choisi parmi les émulsionnants siliconés, les émulsionnants non ioniques dérivés d'acide gras et de polyol, les alkylpolyglycosides et leurs mélanges.

31. Composition selon la revendication 30, **caractérisée en ce que** l'émulsionnant siliconé est choisi parmi les dimethicone copolyols et les alkyl diméthicone copolyols.

32. Composition selon la revendication 30, **caractérisée en ce que** l'émulsionnant est choisi parmi les alkylpolyglycosides représentés par la formule générale (I) suivante :
R-O-(G)x (I)
dans laquelle R représente un radical alkyle ramifié et/ou insaturé, comportant de 14 à 24 atomes de carbone, G représente un sucre réduit comportant de 5 à 6 atomes de carbone, x désigne une valeur allant de 1 à 10, et G désigne le glucose, le fructose ou le galactose.

33. Composition selon la revendication 30, **caractérisée en ce que** l'émulsionnant est choisi parmi les esters d'acide isostéarique et de polyols, les esters d'acide stéarique et de polyols, et leurs mélanges.

34. Composition selon l'une quelconque des revendications 22 à 26, **caractérisée en ce que** l'émulsionnant est présent en une quantité en matière active allant de 0,1 à 20 % en poids et de préférence de 0,5 à 10 % en poids par rapport au poids total de la composition.

35. Composition selon l'une quelconque des revendications précédentes, **caractérisée en ce qu'**elle contient en plus au moins une poudre organique.

36. Composition selon la revendication 31, **caractérisée en ce que** la poudre organique est choisie parmi les particules de polyamide; les poudres de polyéthylène ; les microsphères à base de copolymères acryliques ; les microsphères de polyméthacrylate de méthyle; les poudres de copolymère éthylène-acrylate; les poudres expansées; les poudres d'amidon; les poudres d'aminoacides ; les particules de microdispersion de cire ; et leurs mélanges.

37. Composition selon l'une quelconque des revendications 31 et 32, **caractérisée en ce que** la poudre organique est présente en une quantité allant de 0,1 à 20 % en poids et de préférence de 0,5 à 10 % en poids par rapport au poids total de la composition.

38. Composition selon l'une quelconque des revendications précédentes, **caractérisée en ce qu'**elle contient au moins un additif choisi parmi des agents apaisants, des parfums, des conservateurs, des agents antioxydants, des séquestrants, des gélifiants, des agents de suspension tels que les bentonites et les hectorites, des émollients, des actifs lipophiles ou hydrophiles et leurs mélanges.

39. Composition selon l'une quelconque des revendications précédentes, **caractérisée en ce qu'**elle présente une viscosité à 25°C mesurée avec un appareil du type Rhéomat muni d'un système de mesure Contraves TV, à une vitesse de 200 tours/minutes et après 30 secondes de stabilisation de 0.2 à 7 Pa.s.

40. Utilisation d'une composition selon l'une quelconque des revendications 1 à 39, pour la fabrication d'un produit cosmétique déodorant sous forme de crème distribuée en tube, en roll-on ou sous forme de stick-grille.

41. Procédé cosmétique pour traiter la transpiration humaine, consistant à appliquer sur la surface axillaire une quantité efficace d'une composition selon l'une quelconque des revendications 1 à 39.

42. Procédé cosmétique pour traiter les odeurs corporelles liées à la transpiration, consistant à appliquer sur la surface axillaire une quantité efficace d'une composition selon l'une quelconque des revendications 1 à 35.
